# EUROPEAN PATENT SPECIFICATION

(11) **EP 3 426 136 B1**
(45) Date of publication and mention of the grant of the patent: **14.08.2024**
(21) Application number: 16882616.2
(22) Date of filing: 28.12.2016
(51) Int. Cl.: G16H 20/30, G16H 40/63, A61B 5/0285, A61B 5/318, A61B 5/1455, A61B 5/00, A61H 9/00, A61H 23/00, A61H 23/04, A63B 7/02

(54) **APPARATUS FOR PROMOTING WOUND HEALING**
VORRICHTUNG ZUR FÖRDERUNG DER WUNDHEILUNG
APPAREIL POUR FAVORISER LA CICATRISATION DE PLAIE

(30) Priority: 30.12.2015 US 201514983803
(43) Date of publication of application: 16.01.2019
(73) Proprietor: Syncardon, LLC, Broomall, PA 19008-2524 (US)
(72) Inventor: KHURANA, Vikas, Broomall PA 19008-2524 (US); STEINBERG, Joel, Margate City NJ 08402 (US)
(74) Representative: Sach, Greg Robert
(86) International application number: PCT/US2016/069040
(87) International publication number: WO 2017/117305

(56) References cited:
- US-A- 3 769 974
- US-A- 4 077 402
- US-A- 4 396 010
- US-A1- 2002 173 735
- US-A1- 2011 087 142
- US-A1- 2013 102 939
- US-A1- 2014 066 868

## Description

### FIELD OF THE INVENTION

The present invention relates generally to a medical device for applying negative or variable pressure to a portion of the body and particularly to methods and apparatus for the application of a sequential and gradient pulse wave for treating wounds that are difficult to heal or 'livelihood limiting claudication and/or ischemic rest pain. An integral part of the present invention includes methods to determine blood flow and effects of treatments on tissue.

### BACKGROUND OF THE INVENTION

Impaired circulation, i.e., decreased blood supply to tissue, is a common condition that can arise from several causes. For example, atherosclerosis of arteries as well as diabetic small vessel disease can cause ischemic tissue, leading to pain and/or non-healing wounds, a serious condition that can lead to limb loss. There are other disorders that can have devastating outcomes and can benefit from enhancing the circulation but are not classically related to named vessel-arterial obstruction. These include diabetic and other types of peripheral neuropathy, reflex sympathetic dystrophy, frostbite, chilblains and trauma induced wounds such as those incurred during combat.

It is well known to treat impaired circulation with pressure devices that squeeze the limb, typically by means of an inflatable pressure cuff or similar device wrapped around the limb. The pressure device moves fluid within the treated limb by applying pressure to only a part of the limb. In the prior art, some types of devices are designed to move arterial blood flow downstream. In some prior art, the devices move fluid from tissues from distal portions of the limb to proximal portions, eventually to the trunk of the body, to reduce tissue pressure in the limb so as to allow improved blood inflow. These pressure devices thus perform external, non-invasive compression therapy.

The prior art uses three techniques to apply the pressure, known as waveforms. The first type of waveform is non-sequential and non-gradient, synchronized with the cardiac cycle, and thus pulsatile. The limb compressing component of the device encloses the leg, but not the foot. This art is illustrated by U.S. patent 2,690,174, by Maurice Fuchs. An expandable bladder is placed around the limb and inflated to a certain pressure for squeezing the limb and forcing blood to flow distally (at least supposedly) to the end of the limb. This waveform is simple and basic, and suffers from several drawbacks. First, the design can use high pressures that thus risks discomfort since the device has no specified method to prevent elevated pressure and/or sustained rather than intermittent squeezing. Second, the entire volume of the bladder, a single compartment, is inflated at the same time, and thus has the potential to push fluid both distally and proximally, rather than only to enhance arterial flow distally to diminish ischemia. This design thus has the potential to undermine the very goal of circulation improvement by pushing fluid toward the trunk.

The second type of waveform is also non-sequential and non-gradient, synchronized with the cardiac cycle and thus pulsatile. Its limb squeezing component, however, does cover the foot. This art is illustrated by U.S. patent 3,961,625, by Richard S. Dillon. An inflatable plastic enclosure is placed around the entire limb, including the foot, and inflated to a certain pressure for squeezing the limb and forcing blood to flow distally (at least supposedly) to the end of the limb. Like the first type of waveform described above, it is simple and basic, but also suffers from several drawbacks. Because, like the first type of pump design, it squeezes each limb using a single compartment, its drawbacks stem from that design. In addition, the entire limb, including the foot is encased in the plastic enclosure. Thus, the foot is squeezed along with the rest of the limb, impeding blood flow into the very part of the limb most vulnerable to ischemia, the end, at the foot. This design therefore inherently puts the foot at risk for more ischemia. The enclosure covers the entire limb including the foot. It is therefore not available for inspection to assure lack of adverse consequences of squeezing, such as swelling or cyanosis. It is also thus not available to place a blood flow monitoring device or sensor, to determine if indeed squeezing is improving blood flow or to guide adjustment of timing to optimize compression timing. In addition, this design uses a rubber cuff at the proximal end of the limb enclosure to provide a seal. The seal, if too firm can entrap fluid in the leg, leading to undesired swelling. If the seal is too loose squeezing the limb via a single compartment can allow fluid to exit the limb proximally that is, opposite to the goal of enhancing distal flow, to the ischemic end.

The third type of waveform is prolonged relative to the duration of the cardiac cycle and of modest strength. This art is illustrated by U.S. patent 5,218,954, issued to Paul S. van Bemmelen and marketed as the ArtAssist^{®} as well as other devices described below. These include the ArterialFlow System^{®} by AirCast^{®} and the FM-220^{®} device by FlowMedic^{®}. This design is purported to improve circulation by reducing tissue fluid, thereby allowing for more arterial inflow. This design has not been shown to actually lead to important clinical outcomes such as limb salvage. Furthermore, the form of this design that utilizes foot compression suffers from the same drawbacks as the second type of waveform cited above that squeezes the foot.

The non-sequential waveform device, synchronized with the cardiac cycle, is used in US Pat. No. 4,077,402 to Benjamin et al. A single inflatable compartment is used to squeeze the limb and generate a waveform.

A non-sequential waveform device, synchronized with the cardiac cycle, is used in US Pat. No. 3,961,625, issued June 8, 1976 to Richard S. Dillon. The device depends on an external source of compressed fluid, as a separate device supplied by the user, to facilitate cuff inflation. The limb is encased in a hard outer plastic shell, in which is placed a single inflatable enclosure. Access to the foot to monitor blood flow and inspect skin status is thus denied.

Another non-sequential waveform device, also synchronized with the cardiac cycle, is used in US Pat. No. 6,921,373, issued July 26, 2005 to Myron Z. Bernstein. It is described as a peripheral circulation enhancement system. It times the compression cycle to start at or just after the P wave, to push fluids out into venous structures and back to the heart. With the next heart beat the extremity is now void of some of the fluid, forcing from the heart fresh oxygenated blood, thereby promoting circulation through the heart.

Two patents provide non-cardiac cycle compressions that last seconds. US Pat. No. 5,218,954 to Paul S. van Bemmelen (ACI Medical, Inc.) and marketed as the ArtAssist^{®} uses inflatable cells that first compress the foot and ankle, and, a second later compresses the calf. This action is described as almost completely emptying the veins of the foot, ankle and calf. This action is supposed to facilitate easier flow of arterial blood to the toes and blood-deprived tissues by increasing the arterial-venous pressure gradient. Calf compression starts 1 second after foot and ankle compression, thus providing sequential compression, from the distal to proximal aspect of the limb, in the opposite direction to arterial flow. Because a cuff covers and compresses the foot, blood flow into this most ischemic part of the limb at the end of the arterial tree during compression, which lasts for 4 seconds, is limited. Likewise lack of access to the foot prevents use of inspection and blood flow monitoring to document benefit with treatments. Published reports of benefit range from 30 to 60% and may require several hundred hours of treatments.

Another device, US Pat. No. No. 6,463,934 to Glenn W. Johnson, Jr. et al (ArterialFlow System by AirCast^{®}, now part of DJO Global of Vista, CA) also describes delivery of rapid inflation, graduated sequential compression from the foot to the calf, in reverse direction to arterial flow. The two non-cardiac cycle and prolonged compression cycle devices are reported to enhance arterial flow in part by reducing tissue fluid and/or venous content and thus venous resistance, to improve arterial inflow.

None of the aforementioned devices have shown ongoing established reliable ability to improve circulation and salvage ischemic limbs or promote rapid healing of wounds in studies. They are considered to be investigational by most health insurance systems.

None of the prior art designs have capability to produce a sequential, proximal to distal waveform that mimics and enhance normal physiologic flows and applies negative or variable pressure to wounds and to tissue adjacent to wounds. It is difficult with the existing pressure therapy apparatus to interpret the inputs from PPG signals correctly for selecting the best moment to trigger the compression. We found that the slope (dY/dX) of the PPG curve rises very rapidly at the start of pulse and its highest value works as the trigger and is predictable of the peak.

Moreover, the prior art is replete with references to the difficulties of achieving high resolution, high accuracy signals from EKG apparatus, and thus, the problem of accurately analyzing such signals and of synchronizing physical actions to them is compounded greatly. For example, https://www.boundtreeuniversity.com/print.asp?act=print&vid=1440881 points out many difficulties in obtaining good lead placement and thus good EKG signal quality in a variety of situations. Among the practice points that must be adhered to by a clinician are the following, any one of which, if not followed, may compromise signal quality:
- The ECG machine should be charged and ready for use
- It should be stocked with sufficient paper for the recording - tissues, clinical wipes, alcohol wipes
- Female patients should remove tights and should be offered a cover for their chest once the electrodes and ECG leads are placed and connected
- Male patients with hairy chests may require to have the electrode areas shaved, with permission, to ensure good skin contact
- The skin should be cleaned and dried, according to local protocol, before placing the electrodes
- The patient should be supine on the couch. If this is not possible due to breathless or back problems, then the couch back may be raised and this noted on the ECG.
- After each recording the electrodes and ECG leads should be cleaned with clinical wipes.
- Limb electrodes must be placed on the limbs - not the trunk
- Ideally arm electrodes at the wrist and limb electrodes at the ankles
- In the case of an amputee place the electrode at the point furthest away but ensure that the electrodes on the other limb is positioned at the same level.
https://www.heartelearning.org/labyrinths?id=41220&parent=41219&sessID=1
United States Patent Application 20110004088 teaches,

Accurate placement of the electrodes on the patient's body surface is required to record a useful ECG using an electrocardiograph or patient monitor. The ideal placement of electrodes for a standard ECG is well defined and accepted within the medical industry. However, routine correct placement of the electrodes in the clinical environment is difficult to achieve for several reasons. First, nurses and ECG technicians are frequently not adequately trained or are too inexperienced to accurately locate the attachment points. Moreover, individual physical characteristics vary widely from patient-to-patient. These variations lead to misinterpretation of the "anatomical guideposts" used to locate the proper attachment points. Additionally, patients sometimes have wounds or bandages that preclude access to the patient's body surface at the proper attachment points. Also, attachment of the electrodes to an ECG machine is often accomplished using long individual ECG leadwires. Even if the electrodes are accurately placed on the patient, the leadwires connecting them to the electrocardiograph may be crossed such that signals are switched at the instrument.

Lastly, Chinese patent CN204742712U teaches:
The object of the present invention: to overcome the disadvantages of the prior art, there is provided a detecting ECG patient services, activities within the scope of the hole through the connection piece annular plate electrode via the electrically detecting cardiac patient service may be suitable for patients of different size, to facilitate ECG testing personnel detects, while protecting the private parts of the patient, to avoid embarrassment; by the action of the bushing cloth, to prevent the connection plate or annular plate in direct contact with the patient's body, so that electrocardiographic relatively comfortable wearing patient service; covering the electrode via through the drape, to avoid exposure to the private parts of the patient wearing the ECG is detected when the patient does not detect the ECG service, to avoid embarrassment; clothes just opposite the garment side front shoulder portion connected as a whole, both the clothes and the other side of the shoulder are secured by Velcro, useful for a patient to wear off electrocardiographic patient services, improve the efficiency of wear off; clothes with opposite sides of a plurality of parallel spines Velcro MAO B, the ECG to detect patient service is suitable for patients of different body type.

US 2013/102939 A1 discloses a device for the compression of the limbs of a subject with pressure impulses synchronized with a QRS-complex of ECG.

### SUMMARY OF THE INVENTION

The present invention seeks to provide a novel apparatus for variable pressure therapy that uses a novel waveform, herein called the sequential gradient arterial pulse wave-synchronized waveform, or, abbreviated, the sequential pulse waveform. The sequential pulse waveform is similar to the prior art pulsatile waveforms in that a pulsatile waveform is created. The sequential pulse waveform is also similar to prior art sequential waveforms in that the limb is compressed in different locations in sequence. However, the similarity ends there. Unlike a pulsatile waveform generated by compression of a limb by a single inflatable cuff, the present invention does not generate a single waveform. Rather the waveform is delivered in sequence along the long axis of the limb, from proximal to distal, with each heartbeat, mimicking and enhancing normal arterial flow. Many prior art devices are designed to provide a sequential waveform, from the distal limb to proximal limb, and is generated by prolonged, greater than 1 second, usually several seconds of squeezing. Such art is proposed to decrease venous and interstitial fluid volume of the limb and thus reduce tissue pressure. This reduced pressure is proposed to reduce peripheral vascular resistance to arterial inflow, as the means to enhance arterial inflow.

Such a design mechanism is however, inherently very limited in its ability to enhance arterial inflow, for the following reason. For a reduction of venous and interstitial peripheral resistance to have any appreciable effect on enabling a drawing in of more arterial blood, that reduced resistance or pressure would have to first be transmitted through capillaries to reach their arterial side. But the capillary system through which the reduced pressure would have to be transmitted is a high resistance system. So the ability to transfer the effects of lower pressure on the venous end of the capillary bed, through that bed to the arterial side is very limited. In contrast the present invention does not attempt to purposefully decrease venous resistance as a means to enhance arterial inflow. Of course, any limb compression will inherently provide some reduction in peripheral resistance. Rather, in the present invention sequential waveforms are delivered, proximally to distally, with each heartbeat, via a series of inflatable cells located along the length of the limb, to mimic and enhance natural arterial flow. The maximal pressure in each cell can be adjusted to generate a gradient of compression pressure, greater proximally and lower distally to enhance the natural pattern of arterial blood to flow down a pressure gradient.

The myocardial-generated R wave, as detected by an EKG device, supplies a signal of a consistent event in the cardiac cycle, in this case an electronic signal in the form of an R wave from which to time beginning of each sequential limb compression sequence and cycle. Thus, the present invention enables body part compression in synchrony with the cardiac cycle. Because sequential limb compression occurs rapidly, during the cardiac cycle, again it is designed to mimic and enhance normal arterial flow. Just when during the cardiac cycle limb compression will optimally enhance distal arterial flow cannot be determined based on the timing of myocardial contraction or ventricular systole. This is due to variable rate of flow of blood down the arterial tree upon being pumped out of the left ventricle as modulated by the degree and location of arterial obstruction. The goal of treatment for the ischemic limb is to maximize blood flow reaching the end of the arterial tree, at, e.g., the foot, hand or amputation stump, to improve and heal non-healing tissue such as ischemic tissue. To accomplish this, flow at the limb can be monitored and timing as well as degree of compression of the limb along its length at multiple sites can be adjusted to maximize antegrade flow down the limb and thus delivery of flow distally, to, e.g., the foot.

The present invention incorporates a monitor to detect degree of distal limb blood flow. It also incorporates ability to sequentially squeeze the limb, proximally to distally, and in a pressure gradient fashion, all designed to maximally enhance distal arterial flow. To document healing, the present invention incorporates an infrared detector that generates a thermal image of tissue in a panoramic format. None of the prior art methods or apparatus have the ability to deliver cardiosynchronized sequential or gradient compression from proximal to distal limb aspects to mimic and enhance normal arterial flow or to provide compression in a manner that is guided by distal limb flow to maximize delivery to the most ischemic aspect of the limb or to document tissue healing with infrared or other detection/display methods. In addition, a negative pressure may be combined at a wound site to increase local blood and lymph flows to the site, as well as flow away from the site when variable pressures are applied locally in synchronous fashion with the gradient compression.

Based upon several sources of input (radiology studies, observational deductions, blood flow monitoring technology) the present device in part enhances arterial blood flow downstream likely in part by promoting neoangiogenesis to create new pathways for blood flow as well as overcoming the critical closing pressure of previously closed pre-capillary arterioles, thus opening perfusion through those capillary beds.

The invention is defined in appended claim 1. Preferred embodiments are defined in the appended dependent claims.

### BRIEF DESCRIPTION OF THE DRAWINGS

FIG. 1 is a view of the simplified depiction of the apparatus for applying positive and/or negative pressure to a portion of a patient, constructed and operative in accordance with a preferred embodiment of the present invention.
FIG. 2 is a view of the detailed illustration of apparatus for applying positive and/or negative pressure to a portion of a patient, constructed and operative in accordance with a preferred embodiment of the present invention.
FIG. 3 is a graphic illustration of arterial pulse wave synchronized pressure wave forms used to promote wound healing on a limb, in accordance with a preferred embodiment of the present invention.
FIG. 4 is a graphic illustration of arterial pulse wave synchronized pressure wave forms used to reduce the symptoms of lymphedema in a limb, in accordance with a preferred embodiment of the present invention.

### DETAILED DESCRIPTION OF A PREFERRED EMBODIMENT

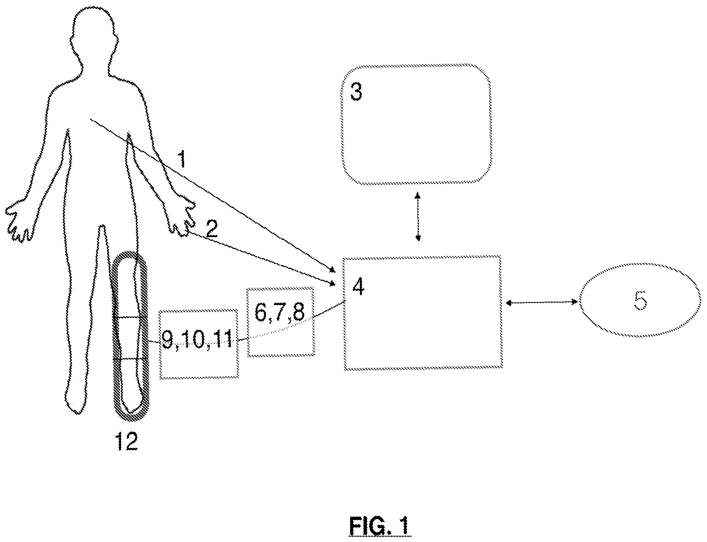

Referring to FIG. 1 in more detail, there is shown a simplified block diagram of apparatus. Below is identification of the components in FIG. 1.
1. EKG signal
2. PPG signal
3. User interface
4. Computing system
5. External database
6. Air Compressor
7. Air Reservoir
8. Inflation valves
9. Conduits
10. Circuitry controlling inflation valves
11. Conduits
12. Inflation Air Cells

FIG. 1 depicts a simplified overall design of the sequential compression system. The system compresses a limb at specific time intervals determined by inputs to the system. Inputs to the system include the EKG (1) and photoplethysmograph (PPG) waveform (2). EKG leads are attached to the body to supply the heart's pulsatile electrical signal, the EKG. At the distal end of the limb where the blood flow increase is desired, a PPG monitor is attached to detect pulsatile blood flow in the underlying tissue. A series of inflatable air cells (12) are wrapped around the limb. Inflation of air cells occurs from fluid filling the cell through fluid traversing from the air (fluid) Reservoir (7) through Conduits (9) through the Inflation Valves (8) through Conduits (11) into the Air Cells (12); Fluid releases from the Air Cells (12) through conduits to Deflation Valves (not depicted). Fluid is generated by an air compressor (10) to maintain fluid under high pressure in the Reservoir (7).

As determined by the device controls, five variables in cell inflation and thus limb compression can be adjusted. The variables are:
(a) delay time: determined by the time interval when, after the R wave occurs, that the most proximal inflation valve opens;
(b) maximal cuff pressure: determined by duration of inflation valve opening as directed by a pressure sensing transducer (not depicted);
(c) duration of cell inflation: determined by time interval during which, after the inflation valve closes, the deflation valve (not depicted) opens;
(d) velocity at which each successive cell inflation begins, beginning proximally, and proceeding distally: determined by the velocity at which each successive inflation valve opening occurs after its most immediate proximal neighboring cell's inflation valve opening occurs; and
(e) sequential air cell compression pressure gradient going distally down the limb: determined by the maximal air cell pressure difference from each proximal cell to its most immediate neighboring cell.

The variables are controlled by one of three options at the user interface (3): a computer-implemented system, operating on computing system (4), which may be a general purpose digital computer programmed on a non-transitory memory medium, that receives input from the EKG and PPG and is designed to maximize PPG waveform size; default settings (found in most patients to maximize PPG size; or manual override by the user (physician, nurse, therapist, etc.).

When a treatment is started, the computing system (4) reads the EKG (1) and PPG (2) inputs and adjusts the five variables for cell inflation to optimize the PPG signal. Alternatively, default settings or manual override can be used. Settings of variables, PPG size and other pertinent information can be broadcast to the physician provider's smart phone, computer, etc. as desired.

The device described above can be used in several types of treatment settings, as follows:

### 1) Hospital environment

In this locale, the device's ease of portability and ease of storage and cleaning to prevent infection are noteworthy assets. The device incorporates the capability to be controlled through multiple screens for the user-interface (3) displaying operational information derived from computing system (4) based upon the requirements of the user administering treatment. Communication with External Database (5) of the device design can be added by incorporating additional measures for communication protocols with the hospital IT systems. This would apply in cases of Electronic Health Record interoperability for patients, data storage for the output of the device usage, tracking the use by multiple types of service providers, appropriately billing in accordance to medical systems coding requirements and the like, and device usage tracking for clinical operations management. Furthermore, cuffs (12) and signal leads (1,2) of the design and any other portions of the device that come in direct contact with the patient or come in contact with diseased areas of the hospital must be designed to resist infectious agents, sterilized for re-use, or disposed of accordingly to eliminate any spread of infection unknowingly.

### 2) Physician specialty office / Private clinic

In this environment, the device's design provides similar beneficial attributes as when used in the hospital environment. These include ease of use and cleaning, multiple methods for control and communication and design for infection control.

### 3) Chronic Disease Management Center

This type of environment includes such facilities as hospital or medical center based outpatient departments, such as physical/occupational therapy, wound care, diabetic, geriatric, podiatry, hyperbaric oxygen facility, rehabilitation and other medical specialty and sub-specialty sections, departments and programs. In these environments, as for other locales of use, the device's design enables ease of use and cleaning, several methods for managing its clinical operation as well as ease of infection control.

### 4) In-home / Caregiver administration

The patient's place of living may be the private home setting, their private apartment, an assisted living facility, long term care center or whatever arrangement has been made to best suit their needs. In these environments the party administrating treatment might be the patient or his/her caregiver, nurse's aide, visiting nurse, physical therapy aide or any one of a number of properly trained medical personnel. The device has the capability to be controlled through multiple screens for the user-interface (3) based upon the requirements of the user administering treatment. The device's default and automatic algorithm capabilities as well as other built in safety features provides a fail-safe method by which almost any person at any education and training level can safely and appropriately use it. Remotely monitoring treatment using telemedicine can be prescribed with the assurance that the physician or other healthcare provider is directing proper and safe administration of treatment with the device. Additional monitoring capabilities and measures including video recording equipment, sensors, and/or any components needed to ensure proper use of the device may be added to the design. Communication with External Database (5) of the device design employs additional measures for communication protocols with the IT systems in cases of Electronic Health Record interoperability for patients, data storage for the output of the device usage, tracking the use by multiple types of service providers, appropriately billing in accordance to medical systems and/or retail system, and device tracking for operations management.

For the above mentioned scenarios, the device may be combined with other treatments for improved blood flow, such as the following:
1) Vasodilation medication, for example alpha blockers: Hydralazine, nitroglycerin, isosorbide mononitrate, sodium nitroprusside PDE5 inhibitors: sildenafil papaverine, minoxidil, Cilostazol, a selective of 3-type phosphodiesterase PDE3 inhibitor, Prostaglandin E1 and counter irritants like capsaicin. These agents dilate the main arteries; this allows for the device to further increase the pre-capillary pressure head and thus enhance downstream capillary blood flow at the extremity with less energy. These agents can be applied to the skin or taken by oral or parenteral route.
2) Combination therapy can be provided with hyperbaric oxygen or other therapies to enhance wound healing in patients with wounds of the extremity including but not limited to gels, growth factors, barrier devices, antibiotics, tissue material, skin transplant, stem cell therapy, etc.
3) Medicated gels at the extremities are methods for vasodilation to increase blood flow; these gels can cause vasodilation but are not effective in reversing infections or wound healing. The device can be used in conjunction with these treatments.
4) Synthetic muscle fibers and other dynamic compression devices can be activated at any point in the compression cycle of the device or before or after treatment. These modalities act as an additive in the localized force allowing for better sequential compression by the device, as they can remove the dependence on air flow based compressions and can provide additional capacity to have multiple pressure pulses during a blood pressure wave.

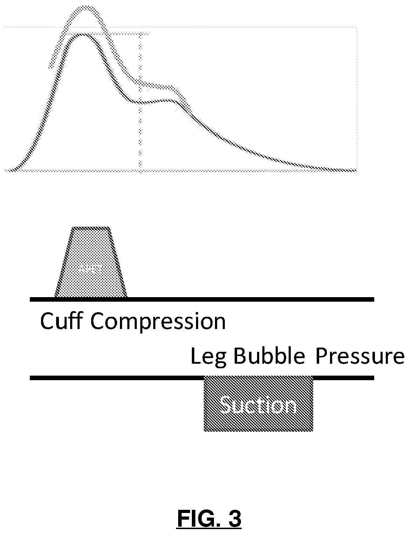

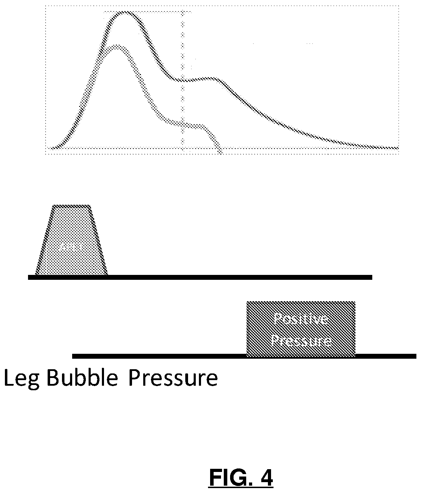

The importance of enhancing normal pulsatile flow as a salient means to facilitate improvement of arterial circulation and overcome ischemia cannot be overemphasized. Given the connectivity of the device's air cells and the number of cells that can be contiguously inflated to enhance pulsatile flow, the limb compressing pressure can be altered in a way to mimic and conform to the above physiological pulse form as shown in the aortic pressure curve. The current method of providing pressure is more rectangular as shown in the image below. The device of the present invention is capable of providing pressure gradients and changes that can be modified to mimic the physiological pulse waveform. See FIG. 3 and 4.

Different types of pulses convey different disease activity. Once a specific pulse is diagnosed the correction can be made in the algorithm to treat the patient to maximize the microcirculation for that condition.

As is clear from the above discussion, the capability provided by the present invention will function as an artificial auxiliary heart to enhance the blood flow at the microcirculation level.

Acupuncture can open the blood vessels allowing for less resistance to blood flow, thus requiring less energy to facilitate blood flow. In patients with borderline cardiac performance like severely impaired left ventricular function, atrial fibrillation or compromised diastolic or valvular function, reduction in resistance to blood flow can facilitate better overall cardiac performance and circulation.

Sensors at key points of the device can provide feedback necessary to deliver higher quality treatment.
a. Vibrations: a vibration sensor can be used to sense the start of the movement of the blood. This may not be the actual bloodflow.
b. Indwelling pressure sensor catheter: there are catheters which can be put into major vascular structures close to the site of treatment which will sense the actual pressure and the feedback from those pressure can help to guide the adjustment in the cuff pressure.
c. Wave form at selected point: the heart pumps the blood in the classic waveform that is critical to mimic physiological functioning. Various points on the waveform if enhanced may have different effect on the ultimate microcirculation of the treatment part. A sensor programmed pickup of the waveform and start compressions based on the waveform or the compressions based on the position of the waveform as predicted by the structure of the previous waveform. Once the change in the pressure has been applied, the waveform changes in a predictable manner. This can be used to further modify and fine-tune the end waveform will have the physiological effect for an individual patient.
d. EKG signal: the electrical activity of the heart travels faster than the actual blood and with current sensors can be picked up from different parts of the body including the extremities, chest or a combination of multiple points.
e. Doppler flow: can detect the actual flow of blood under the senses and alteration of the actual flow by the changes in the pressure can be used to further modify the pressure to a desired goal.

Smart materials are designed materials that have one or more properties that can be significantly changed in a controlled fashion by external stimuli, such as stress, temperature, moisture, pH, electric or magnetic fields. As the field of smart materials develops, materials are available which can stiffen with the passage of electric current. As the electric current can be passed very rapidly with the current electronic circuitry the speed and the options to mimic the pulse wave accurately is significantly enhanced. The use of such material leads to significant approximation to the natural state of pulse. This effect can be used to enhance the treatment potential of the invention.

Tourniquets using the invention to provide compression of the limb between the venous pressure and arterial pressure will engorge the veins without blocking/diminishing the arterial flow. The embodiment can be used for either placement of vascular cannulas or for blood draws.

The entire limb can be encased in the smart material to enhance the removal of excess fluid on edema by compressing in the physiological way to remove fluid without preventing or impeding the arterial or venous blood flow.

There is a significant problem of syncope whenever high gravitational forces are applied to the human body. The blood starts pooling in the dependent portion of the body causing severe circulatory irregularities and can lead to stroke or syncope. This device can be connected to the thighs and arms to prevent excessive or diminished flow to the center of body in case of significant gravitational forces.

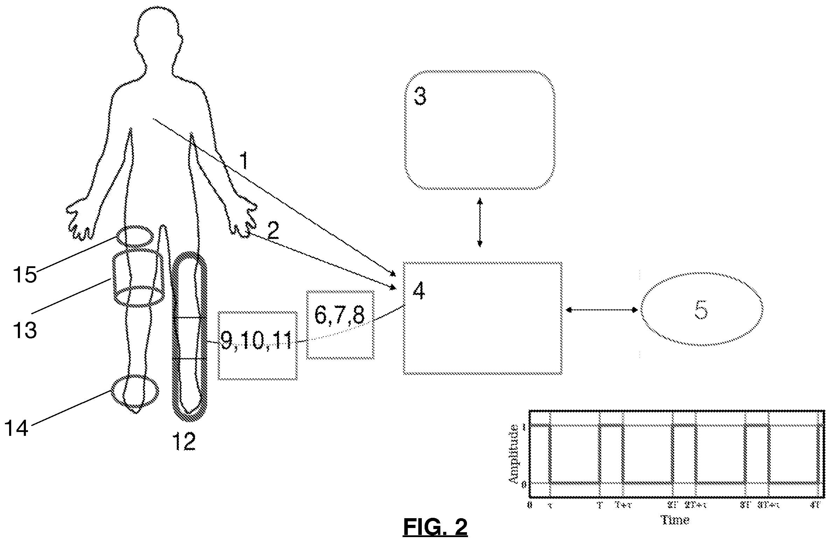

As depicted in FIG. 1 and FIG.2, the inflation system is comprised of the individual components: Air Compressor (6), Air Reservoir (7), Inflation valves (8), Conduits (9), Circuitry controlling inflation valves (10), Conduits (11), Inflation Air Cells (12), and can be replaced with other mechanisms that actuate compression onto the limb in a sequential manner. These include:
1) Nanotechnology of material which stiffens with electrical current or fluid or any other activator where the material would surround the limb to be compressed, have appropriate conduits to an actuator, and have the capability to compress at local areas along the entirety of the limb or area to be compressed.
2) Using the Inflation System of any fluid other than air that can compress the limb.

An alternative embodiment may also combine a sub-atmospheric pressure reservoir and vacuum pump, together with suitable valves and valve actuators with the Inflation System elements already described. By combining these elements, extremely rapid inflation-deflation cycles may be achieved by venting the inflation air cells to the low pressure reservoir, rather than to the ambient atmosphere. Additionally, the sub-atmospheric pressure reservoir may be used in combination with vacuum wound healing dressings to provide wound treatment that overcomes the deficiencies inherent in prior art vacuum wound dressing systems.

Use of the device can be applied to any other body parts outside of the leg including arms to improve blood circulation at the distal ends, to treat, e.g., Raynaud's, frostbite and so forth, and upon the torso to improve blood circulation at any areas with reduced bloodflow, as well as, conceivably, the carotid arteries if compression of trachea can be avoided, so as to treat hair regrowth, eye disorders, and brain diseases and disorders including Alzheimer's.

The device can be applied to one area of the body to treat, heal, or affect other areas of the body. Blood flow improvement in one area can have a parallel affect in the contralateral limb or just allow better overall blood flow to the entirety of the body. This can cause healing in patients in a myriad of conditions including:
1. Hair growth on scalp
2. Increased brain blood flow in age related memory disorders
3. Stroke: selectively compressing carotid artery and preventing pressure on the trachea so the person can continue to breathe
4. Visual loss due to vascular diseases of eye
5. Sinus blood flow to heal sinus infections
6. Carotid sinus pressure or massage can be used to treat hypertension and increase parasympathetic tone/ discharge in the body

The signals and feedback loops are used to determine the starting time within the cardiac cycle of the sequential cell inflation and deflation cycle. The signals and feedback loops can be altered to provide a more accurate starting time of the compression cycle. The R wave of the EKG serves as the starting point to initiate each limb or vessel compression cycle. Other physiological or environmental signals can be used in the system as necessary to provide better information on increasing blood flow at the extremities. While in treatment, a feedback loop using the waveform size generated by the photoplethysmography (Pulse oximetry, tissue PO2 tension or other modes of evaluating tissue perfusion changes), provides evaluation of the effectiveness of that timing and other compression parameters (e.g., duration of each compression) so as to improve blood flow at the target site. Another signal that can be used to optimize the timing and other variables of limb compression is the ratio of PCO2 to PO2 in the blood at the distal end of the limb.

Alternatively, in patients with atrial fibrillation and other rhythm disturbances, there may be poor correlation between EKG and blood flow. In such conditions the plethysmogram reading can be the only loop sensing in milliseconds and sending the signal to activate the pump with a brief delay.

The use of limb EKG leads to provide EKG signal is a significant innovation operationally. It removes the need of making the patient bare chested to place EKG leads and thus make the application of device in convenient open space as compared to a locked space. In addition, it decreases time of person setting up the device and is more convenient for the patient.

### Data Use Strategy

- Use to optimize the time of treatment (eg 1st session 20 min, then 35min and then 30 for 4 sessions, spaced at day 0,2,4,6,7am, 7pm etc)
- Use to optimize the rate of pressure change in given session and later on optimizing opening of blood vessels and prevent damage to vasculature. (There will be an inflection point after which increased pressure will damage the vascular endothelium.) Long term data will feed back into optimizing the pressure details and duration and frequency timing for treatment sessions.
- Short term optimization data will provide the best time to start treatments for new patients, and what standardized or default settings may be optimal.
- At home devices will be based on those setting requiring no input or minimal input from the provider.

Referring to the invention in more detail, the apparatus in accordance with the invention is shown schematically on the block diagram in FIG. 1 and FIG. 2 of the drawings in more detail. Its function and operation is herein described in detail. The apparatus is illustrated in these figures in association with a patient who is shown schematically for the treatment of a wound or ulcer on the lower leg, for example. As will appear more fully hereafter, the patient is electrically connected to the apparatus by way of electrocardiograph electrodes and their conducting wires (1), the patient is mechanically connected by way of a probe (2) of a blood flow measuring instrument which optically detects blood pulse from the skin, in this case a toe, and the patient is pneumatically connected to the apparatus by way of a cuff (12) placed circumferentially around the limb. The cuff (12) contains a series of cells, each adjacent to its immediate neighbor, and each can be partially overlapping the immediately neighboring cell. In practice, cells are assigned sequential numbers. The most proximal cell is located on the proximal thigh of the leg and is designated '12 leg cell 1' (12LC1). The most distal cell, located on the distal calf, is designated, in this figure, 12LC3. The number of cells can be unlimited, to a finite number N, thus, the most distal cell on the limb can read 4LCN. If the limb to be treated is an upper limb, i.e., arm, the most proximal cell, '12 arm cell 1' (12AC1), is located around the proximal arm.

The apparatus comprises means for applying pressure to the selected body part of the patient. Such means comprises a series of inflatable cells contained within a cuff, placed adjacent to each other on the limb. Each cell encircles the body part. The cuff containing the cells is held around the limb by any suitable means, such as by hook and loop or Velcro^{®} strap.

Each cell consists of an inflatable, elastic hollow cylindrical means having an elastic inner wall and a similarly flexible but inelastic outer wall. The cells can take the form similar to a blood pressure cuff structure.

The apparatus is provided with means for supplying air under pressure to the cuff cells, via a series of contiguous conduits, extending from the fluid reservoir, and connecting through to contiguous conduits. A quick connect/disconnect or coupling port may be affixed within the housing wall, and is connected respectively to the air cells.

The above referenced contiguous conduits have four connections. One is the port of a series of solenoid-operated two way valves, inflation valves, with one valve supplying each cell. A positive pressure is supplied to a port of each of these valves by way of the conduits. Each cell is supplied with fluid under pressure by its own inflate valve. The conduit for each cell is connected to a fluid reservoir. The air reservoir is supplied with fluid, as a gas, under pressure by a gas compressor pump.

Another series of valves, deflate valves, one for each air cell, are connected with the conduit system to control flow therethrough, that is, to facilitate air cell deflation. These valves are actuated between open and closed positions by their respective connections to the computer. Pressure transducers sense pressure in each cell via their connectivity to the fluid conduction system. The transducers control energization of the solenoid for inflate valves.

The conduit system is also connected to safety check pressure valves. The control system provides for adjusting delay in cuff inflation from R wave, peak cuff pressure, duration of cuff inflation, velocity of inflation of each bladder in succession and also gradient of peak pressure from proximal to distal cuffs so as to most mimic and enhance normal physiologic blood flow, in the following manner.

The pneumatic system operates as follows:
At the time of initiation of a cycle, the cells, in, e.g., the leg cuff, 4LC1-4LC3 are in a deflated condition so that the pressure transducers sense zero pressure and the transducer switch maintains the inflation valves in the closed position. Since valves are closed, the air under pressure from the reservoir is shut off from going through these valves via the conduit system to their respective air cells (4CL1 through 4CLN). Each limb compression cycle begins with the initiation of the QRS complex from the electrocardiograph at time t0.

Each R wave starts a delay circuit which times out at time t1 at which time valve is actuated to its open position interconnecting conduits. The delay value, time from t0 to t1, is set at a default value, or set per the controller so as to maximize downstream pulse wave as gauged by the PPG wave form amplitude. The goal is to delay onset of cell inflation and thus limb compression so the artificial pulse created by cell inflation and thus limb compression occurs simultaneously with the natural or physiologic pulse, to maximally enhance that physiologic pulse and resultant downstream flow.

The algorithm provides for inflation valves to remain open sufficiently long so that maximal or peak cell pressure is reached to maximize PPG wave form amplitude. The default value of peak cell pressure may be a desired value as shown to maximize PPG size. To accomplish cell inflation, air is applied to cuff 4LC1 through its inflate valve. The pressure transducer allows inflation to continue until the cell 4LC1 has achieved a pressure which opens a pressure transducer switch (not shown), thus actuating supply valve to the closed position and maintaining the cell pressure as determined by the control computer.

Meanwhile, an electronic control circuit that timed out the delay period also started a duration counter at the same time as it actuated deflate valves to their closed position to maintain air cell pressure. The duration times out, i.e., ends, at time t2 whereupon deflate valves are restored to their original open position, thus allowing open access of the air cells under pressure to ambient air and thus allowing ready deflation and rapid exhaustion of the cells 4LC1-N.

The system also provides for adjustment of duration of cuff inflation as follows. Cuff inflation is set at a default duration value. The algorithm is guided by PPG waveform amplitude to increase duration to maximize this amplitude. Duration of inflation is set to a maximum value that will not impede cuff deflation and cuff emptying before the next cardiac cycle begins. Thus, if a longer duration than the default value increases PPG amplitude, it will be used; if not, the algorithm defaults to its original value. So, the algorithm is designed to maximize downstream blood delivery, as determined by the PPG pulse wave amplitude.

Thus, the computer control system, in accordance with the invention comprises a feedback loop in which the cell pressure is measured and this measured cell pressure is allowed to determine how long the filling is to continue.

The controlling system provides to adjust velocity at which each successive cell inflate valve opens to begin that cell's inflation after its most immediate proximal neighboring cell's inflate valve opens. Or cells can be set to inflate simultaneously. A default value is provided for the velocity at which each successive cell inflation valve opens after its most immediate neighboring cell's inflate valve. Feedback from the PPG waveform size drives the algorithm to adjust the velocity up or down, to as high as a default maximum value and down to a default minimum value, or simultaneously. To create a high level velocity, the cells can be in an open system (with output of individual cell connected to atmosphere) or closed system (with output of individual cell connected to one another). The velocity value that leads to maximal PPG size is maintained.

As described above, the pressure wave is modified by interconnectivity of various cells. The various valves can open and close depending on the pressure required to mimic the physiological pulse wave. The valves can be of multiple types, one-way valve, bidirectional valve, open to atmospheric pressure, connected to a sub-atmospheric pressure reservoir, connected to the next cell or connected to multiple cells, these changes will provide a method to control the various pressures and change them to mimic the enhanced physiological pulse wave. This physiological enhancement is critical to improve the microcirculation in the diseased state.

The interconnectivity is one important aspect of the present invention as this may be used to enhance the speed of pressure changes. As these valves release the pressure from one cell to another the changes in the pressure are smoother and do not cause any significant shock to the vasculature thereby preventing further damage. The speed can be altered very rapidly if they are interconnected as the pressure is not required to be brought down to zero or hundred percent for individual cells only and this cuts down the time for enhancement of the next cell by the transmitted pressure from the reservoir. Alternatively, each cell or any subset of cells may be connected to a sub-atmospheric pressure reservoir in order to provide for rapid pressure adjustment, and valves may be modulated to provide smooth transitions that a free of shock.

The current way of providing pressure treatment to enhance circulation is not physiological and is more vertical as shown in the image with on and off options only. This may provide significant shock to the microcirculation leading to more damage than providing any benefit.

The algorithm also provides for adjustment of the gradient of pressure of peak cuff inflation, from the most proximal to the most distal bladder. Thus, the gradient can be set to a value of zero between adjacent bladders or up to a value as desired in mmHg.

Means are provided for controlling the inflation of the cuff by controlling the application of air to the cuff from the pressure supply. Such means comprises a means to actuate inflation valves between their closed positions so no fluid can flow through the valves and there is no communication between the positive pressure supply conduits and an open position in which the valves provide communication between the fluid reservoir and its conduits. The control means also comprises a duration controller function which determines the time period during which the valve drives hold the valve in the open or "inflate" position. Connected to the duration controller function is a delay controller function which initiates operation of the duration controller. The velocity control function determines the velocity with which the inflation valves open to enable filling of each sequential cell from the most proximal cell through 4LCN, with N being the total number of cells, after the inflation valve for the most proximal cell 4LC1 opened to start inflation of that cell. The gradient control function determines the decrease in pressure of each successive cell (4CL2-N) compared to the most proximal cell (4CL1).

The delay controller function, pressure controller function, duration controller function, velocity controller function and gradient controller function are operated in response to various conditions of the patient and the apparatus in accordance with the invention comprises means for sensing these conditions. To this end, there is provided an electrocardiograph function comprising electrode means which includes electrodes attached in a standard Einthoven triangle manner and which sends its signal into an EKG amplifier (not shown). The amplifier is designed in this device to detect in sequence signals from lead positions I, II and III of the standard Einthoven triangle format. The amplifier has an additional function to measure each of these three inputs, from lead positions I, II and III, and to determine which is strongest, that is, provides the highest QRS amplitude. This strongest value, detected, new to this device, is provided as its output. That output is connected by suitable electrical lines to the delay controller function.

There is also provided a blood flow measuring instrument in the form of a photoplethysmograph which uses a probe or any comparable blood flow detecting system attached to the distal part of the limb, e.g., toe or some other location such as a non-healing amputation stump whereas the flow is directly related to the blood flow in an affected body part. The probe or a comparable blood flow detecting device, optically, in the case of the PPG, senses the blood flow and provides an electrical output signal having an amplitude and wave shape corresponding to the blood flow being sensed. The output signal of the probe is connected to the amplifier of the blood flow measuring instrument (not shown), the output of which is connected to a control computer by suitable electrical lines. The signal from the amplifier is also optionally delivered to a display screen which displays an image of the blood flow sensed. The signal from the amplifier is also provided into a circuit that registers its amplitude at specific times as desired, for example, a pre-treatment or initial reading of the pulse wave amplitude, in mm; and at time intervals during a treatment, such as 1) a minute after a treatment is initiated, 2) half way through a treatment session; and 3) just before a treatment ends. The pulse wave registering circuit calculates the percent change in pulse amplitude that is measured at the three times during a treatment and displays that value in the Pulse Wave Change display, item.

The outputs from the EKG amplifier and its R wave selection system and the photoplethysmograph amplifier are connected to the control computer.

The control computer is also provided with output for information display of the electrocardiograph and blood flow pulse signals as well as the percentage of PPG increase during treatment versus pretreatment value. The output also allows for a manual data input screen to control, and/or override the pressure values, timing delay, patient information, pertinent treatment parameters and outcomes

In succeeding cycles of operation, the computer controller function sequentially alters the timing of either the delay circuit or the duration circuit a small amount in one direction. The computer controller function registers the results during each succeeding cycle of operation. If the previous alteration of the timing of the delay controller function or duration controller function results in an increase in the output from the PPG amplifier the next computer controller function alteration is in the same direction. If, on the other hand, the prior alteration results in a decrease in the output from the PPG amplifier, the next alteration is in the opposite direction. In this manner, after several cycles of operation, wherein the computer controller function has been alternately adjusting the delay controller function and the duration controller function, the delay controller function and the duration controller function are finally set to produce optimum results, namely the maximum blood flow in the region being sensed by the PPG probe.

In succeeding cycles of operation, the computer controller function sequentially alters the velocity of sequential cell inflation via its controller function starting at the default value and then moving the velocity a small amount in one direction, faster, and ultimately to simultaneous inflation of all cells. The computer controller function registers the results during each succeeding cycle of operation. If the previous alteration of the timing of the velocity controller function results in an increase in the output from the PPG amplifier the next computer alteration is in the same direction. If, on the other hand, the prior alteration results in a decrease in the output from the PPG amplifier, the next alteration is in the opposite direction, to a slower velocity. In this manner, after several cycles of operation, wherein the computer controller function has been alternately adjusting the velocity controller function, the velocity controller function is finally set to produce optimum results, namely the maximum blood flow in the region being sensed by the PPG probe.

In succeeding cycles of operation, the computer controller function sequentially alters the gradient of pressure in each successive or adjacent cell via its circuit, starting at the default value and then adjusting the gradient in on direction or the other. The computer controller function registers the results during each succeeding cycle of operation. If the previous alteration of the gradient controller function results in an increase in the output from the PPG amplifier, the next computer alteration is in the same direction. If, on the other hand, the prior alteration results in a decrease in the output from the PPG amplifier, the next alteration is in the opposite direction, to a different gradient. In this manner, after several cycles of operation, wherein the computer controller function has been alternately adjusting the gradient controller function, the gradient controller function is finally set to produce optimum results, namely the maximum blood flow in the region being sensed by the PPG probe.

The computer consists of a microprocessor suitably programmed to run the program described above, which is stored therein on a non-transitory storage medium and there is provided suitable switching means to cut the computer out of the control function if desired. This permits the control to be performed either manually or by another control function such as default settings.

There is also provided means for setting the duration controller function to be a fixed fraction of the pulse rate. To this end, the operator is provided with guidelines for setting the duration as a reciprocal of the pulse rate so that, as the pulse rate increases, the duration time is shortened. The option to manually set the duration is provided via an appropriate switch for connecting it into and out of the circuit of the apparatus as desired.

The pressure controller function is provided with a control dial or comparable adjustment capability for the manual setting of the pressure, the delay controller function is provided with a control dial or comparable adjustment capability for the manual setting of the delay time, the duration controller function is provided with a control dial or comparable adjustment capability for the manual setting of the duration time, the velocity controller function is provided with a control dial or comparable adjustment capability for the manual setting of the velocity and the gradient controller function is provided with a control dial or comparable adjustment capability for the manual setting of the gradient.

A typical treatment of a patient by the use of the apparatus in accordance with the invention takes the following course:
After the pressure, delay, duration, velocity and gradient have been set in the apparatus, the apparatus is allowed to run for the length of time determined by the operator, typically an hour. The pulse wave form amplitude percent change register provides a continuous display of the percent change. The value is typically provided as a value to be supplied as a smart phone signal data. The apparatus is typically adjusted to provide the percent value at the beginning of a treatment, half way through the preselected treatment duration and shortly before the treatment ends.

Infrared sensor (15) acquires input of tissue infrared emission. It in turn is connected to geometric optioned platform (not shown) that enables the sensor to be adjusted to conform to the panoramic geometry of the tissue being scanned. At the discretion of the user, a sensor scans tissues of interest. The resultant acquired signal is sent to a transducer (not shown). This transducer displays its acquired signal on a screen and/or supplies the signal for display in an appropriate software/hardware system (e.g., smart phone or computer screen). As depicted on FIG. 2, a blood pressure cuff 13 may provide information to the present invention. Also as shown, a negative pressure cuff 14 may be positioned at a wound site on a limb (shown on the limb not under compression for clarity, but understood to preferably be positioned at a wound site on a limb under compression by cuff 12.

FIG. 3 and FIG. 4 depict time and pressure curves of air cells during a treatment. Time goes from left to right along the abscissa. Except for the QRS complex, at t0, the ordinate depicts pressure in the air cell. The initial event during a treatment is the production, detection and display of the QRS complex, depicted at t0. At a particular time interval after t0, labeled ti1, the inflation valve for the first or most proximal air cell, Cell1, opens, enabling fluid under highpressure to enter that cell, until a desired pressure is achieved. The inflation valve then closes.

At a particular time duration after ti1, the event td1 occurs, when the deflate valve opens and allows fluid in the first air cell to exit, allowing the cell pressure to come to atmospheric pressure. When the next QRS complex occurs, the previously described series of events repeats.

The time delay from t0 to ti1 is determined by one of three methods. The algorithm can determine this value, as required to maximize PPG waveform size. Alternatively the device can use the default settings for this value. And in addition the delay value can be determined manually by the operator. Likewise, P1 the maximal pressure in the most proximal cell can be determined by either of these three methods, by controlling when the intake valve closes. And the duration of cell one inflation, determined by when the deflate valve opens, at td1, can be varied by one of these same three methods.

The next depicted line drawing is the time and pressure curve for cell two, the next most immediate neighboring cell just distal to cell one. The QRS complex in this curve occurs of course at the same time as the QRS complex event depicted in the top curve.

But the time when the inflation valve for cell 2 opens, ti2 occurs can be longer. The time difference between opening of the inflation valve for cell 1, ti1, and opening of the inflation valve for cell 2, ti2, is determined by the velocity with which each successive air cell inflates after its most proximal neighbor.

As with other variables, the velocity value, the speed (in cm/sec) at which each successive cell opens, can be set by the algorithm to maximize PPG waveform size, or to the default value or by operator manual override.

In this depiction, the P value for cell two, P2, is lower than the P value for cell one, P1. The value for P2, the maximal pressure in cell two, is determined by the desired gradient of pressure from the most proximal cell to each successive immediate neighboring cell. And as with other variables, the pressure gradient can be set by the algorithm to maximize PPG size, default value or manual override. Values for variables of each successive cell, cell three and beyond, are determined in the same manner as for cells one and two.

The advantages of the present invention include, without limitation, the following: The ability to apply pressure to the limb to enhance antegrade arterial blood flow. The ability to provide this enhancement with each heart beat and consequent pulse wave. The ability to mimic the natural antegrade flow of arterial flow by providing an extra head of pressure to the pulse wave to push it downstream. The ability to provide flow enhancing pressure to the limb in a sequential fashion, to assure enhancement of flow in the antegrade direction. The ability to provide a gradient of pressure, from proximal to distal, to mimic and enhance the natural flow of arterial blood in the body from the high pressure, generated by left ventricular systole or contraction and aorta elastic recoil, towards the distal parts of the arterial tree. The ability to customize limb compression by adjusting the several cell inflation variables to maximize downstream blood supply. The ability to utilize a blood flow detecting device at the end of the arterial tree to optimize delivery of blood flow to the distal limb. The ability to measure blood flow delivery, as indicated by PPG waveform size, both before and during a treatment, and calculate per cent increase in size during a treatment, to provide objective data on effect of treatment on the circulation. The ability to supply treatment and outcome data to the provider via a broadcast radio signal. The ability to document improvement in wound size and other tissue appearance via detection of tissue infrared emission and thermal display of this information in a panoramic view.

Other physiologic parameters may be measured or used as control parameters for the present invention. Patient-reported pain may be used to moderate or control application of the present invention. Pain in peripheral arterial disease can come from several sources.

Sufficiently severe tissue ischemia often causes pain, usually at the end of the limb, the end of the arterial tree where blood supply is minimal. Indeed, patients often present for treatment because of ischemic pain. Of note, patients with concomitant peripheral neuropathy, common in diabetes, may not experience pain. When pain is a presenting problem with the patient it usually diminishes with successive. Indeed, this improvement becomes a clinical marker of benefits of treatment.

Another potential source of pain is compression of the limb when the device's cuffs inflate and squeeze the underlying soft tissue. Should compression be too firm the patient will complain of pain. Historically maximal cuff pressures above 140 mmHg (2.7 psi) have been found to sometimes produce pain under the cuff.

Enhancement of limb blood flow can lead to pain through various mechanisms. Enhanced blood flow with the consequent increase in blood volume may lead to distention of downstream vasculature and in turn pain. Additionally, in accordance with the Starling-Landis theory of fluid flow between body compartments, increased downstream blood flow may lead to accumulation of interstitial fluid volume and thus soft tissue distention or edema with pain. Distention in or around the delicate vascular infrastructure of capillary beds or pre-capillary arteriole trees can thus become a limiting factor in providing any treatment that enhances limb blood flow.

Of additional concern, compression of the limb by the inflated cuffs may block venous as well as lymphatic return and thus lend towards a build up of distal limb tissue fluid, as edema, that can compromise normal tissue oxygen and nutrient turnover.

Ultimately tolerance of enhanced blood flow will depend on the patient's pain threshold and factors related to the disease process. In addition to the tissue damage that the presence of pain often signals, pain is a noxious sensation, potentially leading to hypo or even hypertension and other adverse systemic effects. Pain is thus a very important factor to be incorporated into the decision-making process of providing treatments and determining optimal as well as safe treatment parameters.

No good method is available to objectively determine pain. Nevertheless, it is imperative that patient input about pain and all other adverse experiences be recorded and used to guide therapy. It is difficult to predict the amount of pain if any that a particular patient may experience. Therefore no standard treatment settings can be used. Before a treatment is begun the patient is instructed that they will feel their limb being squeezed; they are instructed to immediately report any discomfort such as pain. Furthermore, they are instructed how to turn the machine off should they experience any problem. The device is always kept within easy reach of the patient. Patient feedback about pain is incorporated into the adjustment of treatment parameters, including maximal cuff pressure, timing of initiation, duration of pressure. In practice, the rare complaints of pain with treatments have been found to only occur at the beginning of a treatment, at the site of limb compression, and are readily resolved by simply lowering maximal cuff pressure. This approach, using the device algorithm to guide initial treatment settings and then lowering maximal cuff pressure on the rare occasion when pain occurs, serves to maximize blood flow through the limb without compromising tissue integrity or patient comfort. And of course the lower maximal cuff pressure setting is recorded and used for future treatments.

Certainly it is imperative that the treating clinician be aware of the risk of pain and factor that issue in to guide treatments. But, interestingly, rather than the device being at high risk to impart pain, not infrequently a patient may drift off to sleep during a treatment, apparently lulled by the ongoing clicking noises of the device, as the air valves repeatedly open and close with the beat of their heart.

Yet another parameter may be "pinking up", such as may be seen in patients with "diabetic foot", usually a non-healing wound or infection, often on the bottom of the foot, that is without pain because diabetes has damaged pain receptors and nerves. In these situations the clinician depends on tissue appearance, e.g., 'pinking up' as a marker of improvement and as a measure of effectiveness of treatment.

Another embodiment of the present invention uses the measurement of parameters indicative of blood flow to modulate pressures in negative pressure (vacuum) wound treatment (NPWT). It has been reported that prior art NPWT devices may cause dangerous bleeding, shock, low blood pressure, and hematoma. The present invention, by modulating the negative pressure at the wound site in relation to the patient's pulsatile blood flow, avoids or reduces such negative effects, while improving outcomes associated with NPWT.

### Pulse Waveform-Driven Pulse-Enhancing Limb Compression

In this embodiment, each arterial pulse of the distal limb, e.g., the hallux, is detected as the photoplethysmograph waveform. The average time lapse between adjacent pulse segments is calculated. The average starting point of the pulse is calculated. Based on the distance of the most proximal limb cuff from the pulse sensing PPG the expected time of arrival of a pulse at the proximal cuff is calculated. Using that arrival time the timing of the start of limb compression is determined so as to enhance the terminal pulse pressure at the end of the limb. The pulse waveform size is again used as a feedback after every three beats. If the waveform size decreases, the time of onset of proximal cuff inflation is adjusted so that compressions occur at an optimal time to maximize PPG waveform amplitude. The maximal pressure of the cuff can be adjusted electronically or manually by the user.

In an alternate embodiment, each arterial pulse can be detected proximal to the compression site, e.g., the groin, is detected as the photoplethysmograph waveform.

This design configuration-option-provides pulse wave-enhancing-synchronized limb compression without requiring EKG information. This design is simple and easy to use and without a need to connect EKG wires to the patient. It allows very easy home use.

### Distal wound pressure in Combination with Cuff pressure:

A negative pressure source, generated by the air compression system, provides this subatmospheric effect, that is applied to the distal limb during compression treatments. The negative pressure is mediated via a flexible chamber that surrounds the foot. The negative pressure acts to further enhance antegrade, i.e., distal limb blood flow. It accomplishes this by further increasing the pressure gradient along the long axis of the limb. The negative pressure is varied in a cardio synchronous manner. This is a unique feature, not found in other limb compression systems. Various forms of pressure techniques are available but using a available pressure is novel and non obvious.

## Claims

1. An apparatus for improving wound healing by enhancing blood flow in a vertebrate organism's limb comprising:
(a) means for detecting cardiovascular activity of the organism;
(b) means for compressing at least one limb of the organism progressively from a proximal region to a distal region of the limb;
(c) means for triggering the compressing means in timed relation with the detected cardiovascular activity,
wherein the means for compressing at least one vertebrate organism's limb comprises one or more pneumatic devices adapted to substantially surround at least one transverse region of the limb, thereby compressing the surrounded region in response to an increase in pneumatic pressure applied to the device,
**characterized in that**
the means for compressing at least one vertebrate organism's limb comprises a multi-compartment pneumatic device having fluid conduits between adjacent compartments,
wherein the fluid conduits are sized to provide progressive changes in pneumatic pressure within each more distal compartment when such pressure is changed to the adjacent proximal compartment,
wherein each fluid conduit comprises a valve,
wherein each valve changes state in response to a threshold pressure in the adjacent proximal compartment,
wherein the threshold pressure is electrically controllable.

2. The apparatus of Claim 1 wherein the means for detecting the cardiovascular activity further comprises an electrocardiographic apparatus connected to one or more electrodes placed on the skin of the organism.

3. The apparatus of Claim 2 wherein the electrocardiographic apparatus identifies the largest signal from among those detected, and transmits a signal in timed relation with the largest detected signal.

4. The apparatus of Claim 1, wherein the means for compressing at least one vertebrate organism's limb comprises a plurality of pneumatic devices, adapted to apply progressive compression to the limb.

5. The apparatus of Claim 4 wherein a separately controlled source of pneumatic pressure is provided to each pneumatic device.

6. The apparatus of Claim 1 wherein the means for compressing the limb comprises a plurality of hollow soft walled cells that are applied circumferentially around the limb, longitudinally adjacent to each other, along the long axis of the limb, and connected via a fluid conduit to a regulated source of compressed fluid, as well as having connectivity via a fluid conduit to a means to exhaust cell fluid contents in a regulated manner.

7. The apparatus of Claim 3 wherein the means for compressing at least one vertebrate organism's limb is operable to compress the limb in a time interval less than the interval between successive largest detected signals.

8. The apparatus of Claim 1 further comprising means for controlling one or more of the amount of compression, rate of increase of pressure and rate of decrease of pressure applied to the limb.

9. The apparatus of Claim 4 further comprising means for exerting negative pressure at a wound site on the organism's limb.

10. The apparatus of Claim 9 wherein the negative pressure is varied in timed relation to a received signal that is a detected signal indicative of cardiovascular activity.

11. The apparatus of Claim 10 wherein the negative pressure is varied in timed relation to inflation of the immediately proximal limb pneumatic compartment.

12. The apparatus of Claim 10 wherein negative pressure in the most distal limb pneumatic compartment is activated to enter that compartment when positive pressure is changed in the immediately adjacent proximal compartment.

13. The apparatus of Claim 4 further comprising means for exerting negative pressure in successive pneumatic compartments located sequentially along the long axis of the limb, but providing negative pressure from a limb's distal part to its proximal part.

## Patentansprüche

1. Gerät zur Verbesserung der Wundheilung durch Verbesserung des Blutflusses in den Gliedmaßen eines Wirbeltieres, umfassend:
(a) Mittel zum Feststellen der kardiovaskulären Aktivität des Organismus;
(b) Mittel zum Komprimieren mindestens einer Gliedmaße des Organismus von einem proximalen Bereich zu einem distalen Bereich der Gliedmaße;
(c) Mittel zum Auslösen der Kompressionsmittel in zeitlichem Zusammenhang mit der festgestellten kardiovaskulären Aktivität,
wobei das Mittel zum Komprimieren mindestens einer Gliedmaße eines Wirbeltieres eine oder mehrere pneumatische Vorrichtungen umfasst, die geeignet sind, mindestens einen Querbereich der Gliedmaße im Wesentlichen zu umgeben, wodurch der umgebene Bereich als Reaktion auf eine Zunahme des auf die Vorrichtung ausgeübten pneumatischen Drucks komprimiert wird,
**dadurch gekennzeichnet, dass** das Mittel zum Komprimieren mindestens einer Gliedmaße eines Wirbeltieres eine pneumatische Vorrichtung mit mehreren Fächern umfasst, die Fluidleitungen zwischen benachbarten Fächern aufweist,
wobei die Fluidleitungen so bemessen sind, dass sie progressive Änderungen des pneumatischen Drucks in jedem distalen Fach bewirken, wenn dieser Druck in das benachbarte proximale Kompartiment geändert wird,
wobei jede Fluidleitung ein Ventil umfasst,
wobei jedes Ventil seinen Zustand in Reaktion auf einen Schwellendruck im benachbarten proximalen Fach ändert,
wobei der Schwellendruck 1s elektrisch steuerbar ist.

2. Gerät nach Anspruch 1, wobei die Mittel zum Feststellen der kardiovaskulären Aktivität ferner ein elektrokardiographisches Gerät umfassen, das mit einer oder mehreren auf der Haut des Organismus angebrachten Elektroden verbunden ist.

3. Gerät nach Anspruch 2, wobei das elektrokardiographische Gerät das größte Signal unter den festgestellten Signalen identifiziert und ein Signal in zeitlichem Zusammenhang mit dem größten festgestellten Signal sendet.

4. Gerät nach Anspruch 1, wobei das Mittel zum Komprimieren mindestens einer Gliedmaße eines Wirbeltieres eine Vielzahl von pneumatischen Vorrichtungen umfasst, die geeignet sind, ein progressives Komprimieren die Gliedmaße zu bewirken.

5. Gerät nach Anspruch 4, wobei jede pneumatische Vorrichtung mit einer separat gesteuerten Druckquelle versorgt wird.

6. Gerät nach Anspruch 1, wobei das Mittel zum Komprimieren der Gliedmaße eine Vielzahl von hohlen, weichwandigen Zellen umfasst, die in Umfangsrichtung um die Gliedmaße herum, in Längsrichtung nebeneinander, entlang der Längsachse der Gliedmaße angebracht sind und über eine Fluidleitung mit einer regulierten Quelle für komprimiertes Fluid verbunden sind, sowie über eine Fluidleitung mit einem Mittel zum regulierten Absaugen des Zellfluidinhalts verbunden sind.

7. Gerät nach Anspruch 3, wobei das Mittel zum Komprimieren mindestens einer Gliedmaße eines Wirbeltieres so betätigt werden kann, dass die Gliedmaße in einem Zeitintervall komprimiert wird, das kleiner ist als das Intervall zwischen aufeinanderfolgenden größten festgestellten Signalen.

8. Gerät nach Anspruch 1, ferner umfassend Mittel zum Steuern des Ausmaßes der Kompression, der Geschwindigkeit des Druckanstiegs und der Geschwindigkeit des Druckabfalls, die auf die Gliedmaße ausgeübt werden, oder mehrere davon.

9. Gerät nach Anspruch 4, umfassend Mittel zum Ausüben von Unterdruck an einer Wundstelle an der Gliedmaße des Organismus.

10. Gerät nach Anspruch 9, wobei der Unterdruck in zeitlicher Abhängigkeit von einem empfangenen Signal variiert wird, das ein festgestelltes Signal ist, das eine kardiovaskuläre Aktivität anzeigt.

11. Gerät nach Anspruch 10, wobei der Unterdruck im zeitlichen Zusammenhang mit dem Aufblasen des pneumatischen Fachs unmittelbar proximal der Gliedmaßen variiert wird.

12. Gerät nach Anspruch 10, wobei der Unterdruck im pneumatischen Fach der distalsten Gliedmaße aktiviert wird, um in dieses Fach einzudringen, wenn der Überdruck im unmittelbar benachbarten proximalen Fach geändert wird.

13. Gerät nach Anspruch 4, ferner umfassend Mittel zum Ausüben von Unterdruck in aufeinanderfolgenden pneumatischen Fächern, die nacheinander entlang der Längsachse der Gliedmaße angeordnet sind, aber Unterdruck vom distalen Teil der Gliedmaße zu ihrem proximalen Teil liefern.

## Revendications

1. Appareil permettant d'améliorer la cicatrisation des plaies en augmentant le flux sanguin dans le membre d'un organisme vertébré, comprenant :
(a) des moyens permettant de détecter l'activité cardiovasculaire de l'organisme ;
(b) des moyens pour comprimer au moins un membre de l'organisme progressivement d'une région proximale vers une région distale du membre ;
(c) des moyens pour déclencher les moyens de compression en relation temporelle avec l'activité cardiovasculaire détectée,
dans lequel les moyens de compression d'au moins un membre d'un organisme vertébré comprennent un ou plusieurs dispositifs pneumatiques adaptés pour entourer sensiblement au moins une région transversale du membre, comprimant ainsi la région entourée en réponse à une augmentation de la pression pneumatique appliquée au dispositif,
**caractérisé en ce que**
le moyen de compression d'au moins un membre d'un organisme vertébré comprend un dispositif pneumatique à compartiments multiples comportant des conduits de fluide entre les compartiments adjacents,
dans lequel les conduits de fluide sont dimensionnés pour fournir des changements progressifs de pression pneumatique dans chaque compartiment plus distal lorsque cette pression est modifiée vers le compartiment proximal adjacent,
dans lequel chaque conduit de fluide comprend une valve,
dans lequel chaque valve change l'état en réponse à une pression de seuil dans le compartiment proximal adjacent,
dans lequel la pression de seuil est contrôlable électriquement.

2. Appareil selon la revendication 1, dans lequel les moyens de détection de l'activité cardiovasculaire comprennent en outre un appareil électrocardiographique connecté à une ou plusieurs électrodes placées sur la peau de l'organisme.

3. Appareil selon la revendication 2, dans lequel l'appareil électrocardiographique identifie le signal le plus important parmi ceux détectés et transmet un signal en relation temporelle avec le signal le plus important détecté.

4. Appareil selon la revendication 1, dans lequel les moyens de compression d'au moins un membre d'un organisme vertébré comprennent une pluralité de dispositifs pneumatiques, adaptés pour appliquer une compression progressive au membre.

5. Appareil selon la revendication 4, dans lequel une source de pression pneumatique contrôlée séparément est fournie à chaque dispositif pneumatique.

6. Appareil selon la revendication 1, dans lequel les moyens de compression du membre comprennent une pluralité de cellules creuses à parois souples qui sont appliquées de manière circonférentielle autour du membre, longitudinalement adjacentes les unes aux autres, le long de l'axe long du membre, et reliées via un conduit de fluide à une source régulée de fluide comprimé, ainsi qu'ayant une connectivité via un conduit de fluide à un moyen pour évacuer le contenu de fluide cellulaire de manière régulée.

7. Appareil selon la revendication 3, dans lequel les moyens de compression d'au moins un membre d'un organisme vertébré sont capables de comprimer le membre dans un intervalle de temps inférieur à l'intervalle entre les signaux détectés successifs les plus importants.

8. Appareil selon la revendication 1 qui comprend en outre des moyens permettant de contrôler un ou plusieurs de la quantité de compression, du taux d'augmentation de la pression et du taux de diminution de la pression appliquée au membre.

9. Appareil selon la revendication 4 comprenant en outre des moyens permettant d'exercer une pression négative au niveau d'un site de blessure sur le membre de l'organisme.

10. Appareil selon la revendication 9 dans lequel la pression négative varie en fonction du temps du signal reçu qui est le signal détecté pour l'activité cardiovasculaire.

11. Appareil selon la revendication 10 dans lequel la pression négative varie en fonction du temps imparti au gonflage du compartiment pneumatique du membre immédiatement proximal.

12. Appareil selon la revendication 10 dans lequel la pression négative dans le compartiment pneumatique du membre le plus distal est activée pour entrer dans ce compartiment lorsque la pression positive est modifiée dans le compartiment proximal immédiatement adjacent.

13. Appareil selon la revendication 4 qui comprend en outre des moyens pour exercer une pression négative dans les compartiments pneumatiques successifs situés séquentiellement le long de l'axe long du membre, mais exerçant une pression négative de la partie distale du membre vers sa partie proximale.
